# EUROPEAN PATENT SPECIFICATION

(11) **EP 2 219 610 B1**
(45) Date of publication and mention of the grant of the patent: **01.05.2019**
(21) Application number: 08853661.0
(22) Date of filing: 26.11.2008
(51) Int. Cl.: A61K 9/14, A61K 9/16, A61K 31/165

(54) **COMPOSITIONS CONTAINING CAPSAICINOIDS**
CAPSAICINOIDE ENTHALTENDE ZUSAMMENSETZUNGEN
COMPOSITIONS CONTENANT DES CAPSAÏCINOÏDES

(30) Priority: 26.11.2007 HU 0700755
(43) Date of publication of application: 25.08.2010
(73) Proprietor: Pannonpharma Gyógyszergyártó Kft., 7720 Pécsvárad (HU); Szolcsányi, János, 7621 Pécs (HU); Mózsik, Gyula, 7624 Pécs (HU); Perjési, Pál, 7634 Pécs (HU); Past, Tibor, 7623 Pécs (HU)
(72) Inventor: SZOLCSÁNYI, János, H-7621 Pécs (HU); MÓZSIK, Gyula, H-7624 Pécs (HU); PERJÉSI, Pál, H-7634 Pécs (HU); PAST, Tibor, H-7623 Pécs (HU)
(74) Representative: Török, Ferenc
(86) International application number: PCT/HU2008/000136
(87) International publication number: WO 2009/068922

(56) References cited:
- WO-A1-98/56356
- US-A- 4 681 897
- US-A1- 2003 170 181
- ABDEL SALAM O M E ET AL: "Studies on the effect of intragastric capsaicin on gastric ulcer and on the prostacyclin-induced cytoprotection in rats" PHARMACOLOGICAL RESEARCH, ACADEMIC PRESS, LONDON, GB, vol. 32, no. 4, 1 October 1995 (1995-10-01), pages 209-215, XP004902646 ISSN: 1043-6618 cited in the application

## Description

The invention relates to a solid pharmaceutical composition (e.g. capsule or tablet) that contains, as active agents, capsaicinoids in low doses and one or more non-steroidal anti-inflammatory components (Cyclooxygenase-1/Cyclooxygenase-2: COX-1/COX-2 inhibitors), and which can be administered orally and has a local effect; and to a procedure for preparing same. The composition is capable of diminishing or protecting against the gastro-intestinal side effects that may occur in short or long term therapy made by the use of orally administered non-steroidal anti-inflammatory drugs.

### Prior Art

It is well known that inhibition of COX-1 and/or COX-2 can be achieved through the application of non-steroidal anti-inflammatory and pain-killing drugs (whose widely used English acronym is NSAID such as aspirin, diclofenac, ibuprofen), which have, however, negative gastro-intestinal side effects (i.e. in the stomach and the intestines). The COX-inhibitors may cause bleeding, mucosal lesions, gastric or intestinal ulceration, even massive hemorrhages. Earlier solutions have already been suggested to counteract these side effects, for example, a COX-inhibitor alongside with a gastric acid secretion suppressor (e.g. a proton pump inhibitor, such as omeprazole or rabeprazole), occasionally, a COX-inhibitor together with a prostaglandin derivative. In traditional internal medicine, to prevent the gastro-intestinal side effects of NSAID, proton-pump inhibitors are in general use; these compounds are able to change the functioning of the efferent fibers of the autonomic nervous system. But the use of prostaglandin derivatives to prevent the side effects of NSAID has not gained ground as the latter may cause diarrhea and, in pregnant women, even spontaneous abortion.

The inventors proved the above effects in animal studies published earlier when it was shown in experiments run on rats that gastric ulceration and other side effects were less likely to develop when aspirin combined with capsaicin were administered at the same time (OM. Abdel Salam, Gy. Mózsik, J. Szolcsányi: Studies on the effect of intragastric capsaicin on gastric ulcer and on the prostacyclin-induced cytoprotection in rats. Pharmacol. Res. 1995; 32:209-215; P. Holzer, MA. Pabst, IT. Lippe: Intragastric capsaicin protects against aspirin-induced lesion formation and bleeding in the rat gastric mucosa. Gastroenterology. 1989;96:1425-1433).

In other studies carried out with human volunteers, it was possible to show that capsaicin - administered through a gastric sound - eases the bleeding in the stomach caused by ethanol and indomethacin while at the same time it inhibits the secretion of gastric acid (Gy. Mózsik, J. Szolcsányi, I. Rácz: Gastroprotection induced by capsaicin in healthy human subjects. M. Gy. World J. Gastroenterology, 2005; 11:5180-5184).

Although one can find a patent that deals with the inclusion of capsaicin in a pharmaceutical composition to be taken orally (Canadian Patent No. 2447807: "Abuse resistant pharmaceutical composition containing capsaicin"), but there the introduction of capsaicin has a rather special and totally different goal from ours, namely, the prevention of drug abuse (i.e. the thwarting of the use of opiates - oxycodone, hydromorphone and oxymorphone - for non-pharmaceutical purposes). The aims of present invention have no connection whatsoever with drug abuse.

Neither can the invention be connected with the weight-reducing effect of capsaicin, which problem is dealt with in US Patent No. 20060240125 as well as in several scientific papers (A. Belza, E. Frandsen and J. Kondrup: Body fat loss achieved by stimulation of thermogenesis by a combination of bioactive food ingredients: a placebo-controlled, double blind 8-week intervention in obese subjects. Int. J. Obesity. 31: 121-130, 2007).

In US Patents Nos. 4,681,897 and 4,812,446, analgesic combinations are described where capsaicin or a capsaicin analogue is used together with a NSAID-type painkiller. The mass ratios of the NSAID component to the capsaicin or analogue are 10:1 - 1:10. In the examples, the quantity of capsaicin or its analogue is 30-120 mg in a dosage unit, and, in general, the quantity of capsaicin or its analogue is many times the higher (as a minimum, as much capsaicin as the NSAID component is used, but often the former is present in a quantity four times higher as the latter).

Hungarian Patent No. 225,044 describes a topical pharmaceutical composition that contains nimesulid as well as an extract of green peppers and capsaicin as auxiliary materials. The invention is concentrated on the packaging of nimesulid with glyceryl-monooleins, where the advantageous formulation is a gel.

WO 98/56356 provides a composition releasing the active agent in the gastrointestinal tract between the stomach and the rectum of the mammal, therefore the aim of this disclosure is different.

The article of Abdel Salam Ome et al (Pharmacological Research, vol 32, no. 4, October 1, 1995, pages 209-215) discloses the combination of NSAID and capsaicin, however, there is no indication that any carrier is used besides said combination.

US 2003/170181 mentions guar gum as a carrier effective for colonic delivery, so this field differs from the same of the present invention.

### Definition of the Aim

Although it is a known scientific recognition that capsaicinoids may be capable of preventing some of the gastro-intestinal side effects accompanying the use of non-steroidal anti-inflammatory drugs, there is, however, neither commercially available oral composition is known nor such is found in the literature (with the above-mentioned exceptions which are totally different from the goal and recognition of the present invention) that contains one or more non-steroidal anti-inflammatory agents accompanied by small dosages of capsaicinoids, where the basic function of the latter is to prevent the undesirable gastro-intestinal side effects of the NSAID-type active agents.

One of the reasons for this state of affairs is probably that the phenomenon has only recently been recognized, while another reason may be the question of the most effective dosage of the capsaicinoids. As the capsaicinoids are practically insoluble in water, their formulation requires special conditions.

Therefore, the aim of the invention was to develop a solid pharmaceutical composition that contains capsaicinoids and (one or more) non-steroidal anti-inflammatory drugs (from now on indicated by the acronym NSAID) which can prevent a part of the gastro-intestinal side effects occurring during long-term use of the latter and which may be administered orally and has a local effect.

Several new findings have contributed to the development of the invention, where the combined utilization of them - which is not obvious to a skilled professional - ensured the excellent pharmacological properties of the composition.

The inventors perceived that the joint application of NSAID and capsaicinoids was capable of efficaciously preventing the side effects in the stomach and the intestines caused by the NSAID (Gy. Mózsik, OME. Abdel Salam, J. Szolcsányi: Capsaicin Sensory Nerve in Gastric Mucosal Damage and Protection, Akadémiai Kiadó, Budapest, 1997).

An important part of the perception was that the desirable protective effect of the capsaicinoids was such a local effect of the nervous system which has an optimal dosage, whereas high concentrations had an opposite effect (where too low concentrations remaining ineffective). In our experiments it was found that the capsaicinoids prevent the side effects or damage in the stomach and the intestines caused by the non-steroidal anti-inflammatory drugs through stimulating the heat- and chemosensitive fibers of the afferent nervous system, and that this mechanism - quite surprisingly - is effective with small doses while, at the same time, a significant portion of the side effects is eliminated. Given in large doses (above about 10,000 µg = 10 mg per dosage unit), the capsaicinoids damage these fibers.

It is also known that the capsaicinoids absorbed from the intestines have systemic effects through the central nervous system (CNS), which, however, are unfavourable from the point of the protective efficacy. Animal experiments have shown that administration of larger doses has a CNS effect, which may manifest in diarrhoea, sensory disturbance, respiratory problems in insubstantial cases, but in severe cases respiratory paralysis may occur. The undesirable side effects are described in the following publication: Gy. Mózsik, Á. Vincze, J. Szolcsányi: Four response stages of capsaicin-sensitive primary afferent neurons to capsaicin and its analog: Gastric acid secretion, gastric mucosal damage and protection; J. Gastroenterol. Hepatol. 2001 16: 1093-7). The doses that begin to show the CNS effects described above are at least ten times higher than the small doses recommended by the inventors (that is, 100-1200 µg for a patient a day), while elsewhere even doses as high as a thousand fold are known (e.g. 1-2 g per experimental animal). It must also be emphasized that the mean value of the dose range found advantageous in our experiments (about 400 µg = 0.4 mg) is less than a hundredth (about 1/200) of the mean value of the dosage range (90 mg) used in the Patents No. US 4,681,897 and 4,812,446 mentioned above.

So far, the capsaicinoids' protective effect on the stomach when given in capsules or in solid form has not been proven. This is an important fact because the capsaicinoids exert their protective effect on the gastro-intestinal side effects caused by NSAIDs through a localized event, that is a function of the low concentrations released locally from the formulated composition. Should the concentration affecting the mucous membrane of the stomach be higher, the side effects caused by the NSAIDs cannot be prevented by capsaicinoids.

It is an important observation, therefore, that a satisfactory protective effect can only be achieved if the active agent supplied orally is kept on the surface to be protected (e.g. the gastric mucosa) dispersed uniformly and persistently. So, in developing the capsaicinoid-bearing compositions, several formulation-related and pharmaceutical-technological problems had to be overcome. It should be noted that, during the elaboration of the technology, not only pharmaceutical-technological approaches were used but the production technology of columns used in gas chromatography was also taken advantage of in realizing the even dispersal of the active agent.

### Summary of the invention

The aim set was achieved through developing a pharmaceutical composition of a solid formulation that can be administered orally, and which is capable of decreasing or protecting against the gastro-intestinal side effects caused by non-steroidal anti-inflammatory drugs taken orally. This composition contains capsaicinoids in small doses, one or more non-steroidal anti-inflammatory drugs, as well as a special vehicle that ensures its uniform distribution on the mucous membrane while, at the same time, helps to keep up the special, mucosa-protective concentration for a long term.

The aspects and the advantageous embodiments of the invention are as follows:
1. A combined solid, oral pharmaceutical composition for use in diminishing or protecting against the gastro-intestinal side effects of non-steroidal anti-inflammatory agents administered orally in a therapy, which contains capsaicinoid active agent in a dose of 100 to 1200 µg per dosage unit, one or more non-steroidal anti-inflammatory active agent, and one or more usual pharmaceutical auxiliary materials, characterized in that the composition comprises the active agent and other ingredients in a vehicle system which comprises one or more bioadhesive polymer(s) selected from the group comprising guar gum, gelatin, hydroxypropyl methyl cellulose or any mixtures thereof.
2. Pharmaceutical composition for use according to point 1 above, wherein the bioadhesive polymer vehicle is guar gum.
3. Pharmaceutical composition for use according to above point 1 or 2, wherein the non-steroidal anti-inflammatory agent, which is applied in usual dose, is diclofenac, preferably in 25 to 75 mg per dosage unit, or aspirin, preferably in 50 to 500 mg per dosage unit, or naproxen, preferably in 250 to 500 mg per dosage unit.
4. Pharmaceutical composition for use according to any of above points 1 to 3, wherein the dose of capsaicinoid is 200 to 800 µg per dosage unit, preferably 400 µg per dosage unit. Advantageously the composition is administered at least once but at most three times a day.
5. Pharmaceutical composition for use according to any of above points 1 to 4, which contains as usual vehicle talc, micro-crystalline cellulose, C₄₋₇ sugar or sugar alcohol, e.g. mannitol, or any mixtures thereof.
6. A combined, solid, oral pharmaceutical composition for use in diminishing or protecting against the gastro-intestinal side effects of orally administered non-steroidal anti-inflammatory agents in therapy, which comprises in a combined packaging, preferably in a twin blister
   A) a separate, first pharmaceutical composition, preferably a tablet or capsule, which contains one or more non-steroidal anti-inflammatory active agent(s), together with usual pharmaceutical auxiliary materials and/or carriers; and
   B) a separate, second pharmaceutical composition according to any of above points 1 to 5 without non-steroidal anti-inflammatory active agent.

### Detailed description of the invention

As it will have been seen from the above, the protection of the capsaicinoid active agent against oxydation was resolved in two different ways (not part of the claimed scope alone). In the first version, an oily carrier (e.g. triolein also containing oleic acid and esters of fatty acid), in the other, a method of molecular enveloping was used. To accomplish the molecular enveloping, coronaethers or cyclodextrins, more preferably substituted alpha-, beta- or gamma-cyclodextrin, or yet more preferably beta-cyclodextrin may be used.

A further advantage of the molecular enveloping is that the active agent, which is otherwise insoluble in water, could be made uniformly distributed even in an aqueous phase. Utilizing either the molecular complex of the active agent, or the solution/suspension of the active agent formed with the oily carrier, the usual tablets can be formed (using usual carrier, e.g. talc) which will contain the active agent uniformly.

According to the invention, to avoid the undesirable high local concentrations as well as to achieve a persistent and ideal low concentration on the mucous membrane, a bioadhesive vehicle system was used composed of one or more bioadhesive polymers.

In a preferred embodiment the above-mentioned methods producing the positive effects are combined. For example, the active principle can be stabilized by an oily carrier or as a cyclodextrin complex, and this stabilized version is dispersed in the bioadhesive vehicle, which ensures the uniform local distribution of the desired dose.

The bioadhesive polymeric vehicle is selected from the following group: guar gum, gelatin, hydroxypropyl methyl cellulose or any mixture of them.

The non-steroidal anti-inflammatory agent in the pharmaceutical composition can be any known and herein mentioned NSAID, preferably applied in usual dose, preferably aspirin, preferably in 50 to 500 mg per dosage unit, diclofenac, preferably in 25 to 75 mg per dosage unit, or naproxen, preferably in 250-500 mg per dosage unit.

The "small dose" of capsaicinoids in the pharmaceutical composition means that the capsaicinoids are present as 100 to 1200 µg per dosage unit, preferably 200 to 800 µg per dosage unit, and more preferably 400 µg per dosage unit, and the composition should be taken at least once but no more than three times a day.

The oily carrier mentioned above (not part of the claimed scope alone) exerts an antioxidant effect on the active agent and it is selected preferably from the following group: C₂₋₅ monohydric or higher polyhydric alcohols, fatty acids, oleic acids, esters of C₂₋₅ monohydric or higher polyhydric alcohols with C₆₋₁₉ aliphatic carboxylic acids, preferably esters or mixed esters of glycerol with oleic acid, palmitic acid or stearic acid, sunflower oil or re-esterified form of it.

The pharmaceutical composition may also contain one or more inorganic or organic inert carrier, preferably talc, cellulose having a proper grain size in a micro-crystalline form, a C₄₋₇ sugar or sugar alcohol, e.g. mannitol having a proper grain size.

The composition may be used to treat or prevent the following conditions: inflammatory conditions, like gastritis, helicobacter positive or negative clinical appearances; gastro-intestinal inflammatory conditions, like a precancerous state, tumorous states accompanied with significant increase of COX2 activity; conditions accompanied with increasing level of free oxygen radicals; irritable intestinal syndrome and chronic abdominal pain syndrome; cardiovascular diseases; stroke; inflammatory diseases of the organs of locomotion, other inflammatory conditions of the upper or lower limbs. The combined oral composition formulated as described above can diminish or prevent the gastro-intestinal side effects of the non-steroidal anti-inflammatory drugs.

The compositions according to the invention, first of all those containing aspirin, may be administered to patients after a cardiac infarct or prior to an anticipated stroke or a vascular catastrophe in a long-term preventive treatment to protect against blood clotting; in these cases the composition diminishes or prevents the gastro-intestinal side effects accompanying the use of non-steroidal anti-inflammatory drugs.

In an alternative embodiment the solid, oral pharmaceutical compositions - which may be used to diminish or prevent the gastro-intestinal side effects of the short- or long-term oral administration of non-steroidal anti-inflammatory agents - comprises in two separate compositions, preferably in a twin blisters the non-steroidal anti-inflammatory active agent on the one hand, and on the other hand, the capsaicinoids formulated as discussed above. The first composition of the pair - preferably a tablet or capsule - comprises one or more non-steroidal anti-inflammatory agents in the usual therapeutic doses together with usual auxiliaries and/or carriers, preferably 25 to 75 mg diclofenac per dosage unit, or preferably 50 to 500 mg aspirin per dosage unit, or 250 to 500 mg naproxen per dosage unit. The separate second composition includes the above-detailed general and preferred embodiments, with the difference that it does not contain non-steroidal anti-inflammatory agent, but comprises a capsaicinoid as active agent in 100 to 1200 µg per dosage unit, preferably in 200 to 800 µg per dosage unit, more preferably in 400 µg per dosage unit, embedded in the vehicle system according to the invention.

In another possible realization the two separately formulated active agents are packaged together - in a powdered or granulated form - in the same packaging unit, practically in a common tablet or capsule.

### Active agents

In the description and the claims, the terms "capsaicinoid" or "capsaicinoids" mean, on the one hand, all the known capsaicin allotropes and derivatives (first of all, the seven main known capsaicin homologues and three known capsaicin analogues) one by one separately, as well as the synthetic capsaicinoids, and, on the other hand, a mixture of any ratio of all the mentioned active agents. The capsaicinoid in general use is actually a purified vegetable extract that contains a defined mixture of the capsaicin allotropes and derivatives. Among these, capsaicin itself is of outstanding importance (8-methyl-N-vanillyl-trans-6-nonenamide), and the dihydrocapsaicin and the nordihydrocapsaicin. It is noted here that, in patent applications and in the scientific literature - either in animal experiments or in human observations -, the word "capsaicin" is usually mentioned even in cases where, in actual fact, capsaicinoids (one or a mixture of more) were used.

Capsaicin itself or a purified natural capsaicinoid extract may be applied preferably, where the total amount of the capsaicinoids is min. 90%; and with reference to the total amount of the capsaicinoids, the ratio of capsaicin / dihydro-capsaicin / nordihydro-capsaicin varies between about 45 : 40 : 10 and 65 : 28 : 7; whereas the ratio of all the capsaicinoids other than capsaicin and dihydro-capsaicin is not higher than 15%.

In the composition the small-dose capsaicinoid is present as 100 to 1200 µg per packaged dosage unit, preferably 200 to 800 µg packaged dosage unit, more preferably 400 µg packaged dosage unit, and they are administered either separately or combined with the non-steroidal anti-inflammatory agent which is included in the usual therapeutic dose.

The non-steroidal anti-inflammatory drug (NSAID) included in the composition may be selected from among the known NSAIDs (COX-inhibitors) which can be - in a non-restrictive sense - salicylates, e.g. aspirin, amoxypirin; aryl alkane acids, e.g. diclofenac, aceclofenac, tolmetin; 2-arylpropionic acids, like ibuprofen, carprofen, ketoprofen, naproxen; N-aryl antranil acids, e.g. mefenamic acid; a pyrazolidine derivatives, e.g. phenylbutazone; oxycams, e.g. pyroxicam and the tenoxicam, in free form or as any pharmaceutically acceptable salt formed with any acid or base.

### Oily carriers (not part of the claimed scope alone)

As the capsaicinoids are practically non-soluble in water, but the composition must be capable of distributing locally and evenly on the mucous membrane, the desired effect may be achieved through taking up the active agent in an oily carrier and dispersing it on the large, inert surface of the inert carrier. The oily carrier can be selected from the following group: C₂₋₅ monohydric or higher polyhydric alcohols, fatty acids, oleic acids, esters of C₂₋₅ monohydric or higher polyhydric alcohols with C₆₋₁₉ aliphatic carboxylic acids, preferably esters or mixed esters of glycerol with oleic acid, palmitic acid or stearic acid, sunflower oil or re-esterified form of it. The amount of the oily carrier to the vehicle preferably vary between 2 to 20 % by mass, more preferably 10% by mass. It is more preferred if the oily carrier contains unsaturated double bonds (oleic acid, glycerol trioleate) because thus it can protect the active agent against oxidation, which improves the stability and extends the shelf life of the product. In the process of its formulation, attention must be paid to the sensitivity of the capsaicinoids, and measures must be taken to prevent oxidation and hydration.

For the joint dissolution of both the oily carrier and the capsaicinoids, a volatile solvent can be selected from the following group: C₂₋₄ alcohols, preferably ethanol, ethers formed from these alcohols, conveniently diethyl ether or diisopropyl ether, esters formed by these alcohols and C₂₋₄ aliphatic carboxylic acids, conveniently ethyl acetate, or a suitable mixture of the volatile solvents mentioned. The amount of the volatile solvent (or mixture of solvents) to the vehicle may vary between about 50 to 800% by mass.

The capsaicinoids dissolved in a suitable mixture of the oily carrier and the volatile solvent are brought into contact with the surface of the vehicle by impregnation or vaporization, taking care to produce a uniform layer. The volatile solvent (or mixture of solvents) is then removed by vacuum thin-film evaporation at 20 to 80 °C, conveniently at 40 °C, in a vacuum of -30 to -95 %, conveniently of -60%. If desired, vacuum evaporation can further be used with the above conditions to remove the remaining volatile solvent, where the vacuum should be kept near to the higher limit given above.

### Usual vehicles

As a first step of formulation, the capsaicinoid active agent can be applied to an inert, organic or inorganic vehicle [which can be talc, micro-crystalline cellulose having a suitable grain size, a C₄₋₇ sugar or sugar alcohol, e.g. mannitol having a proper grain size] utilizing a volatile solvent (or a mixture of such), always looking out for a uniform distribution. Next, the volatile solvent is removed from the impregnated vehicle by vacuum-evaporation, and, from now on, the formulation is carried on with the impregnated vehicle material.

### Bioadhesive polymer vehicles

The objective that the capsaicinoid is capable of exerting its lasting protective effect locally at the desirable locality, can be achieved by using - besides the customary auxiliary materials - a bioadhesive polymer vehicle that will adhere well on the mucous membrane.

The capsaicinoid active agent should be evenly dispersed by a wet process in a known bioadhesive polymer vehicle, e.g. guar gum, gelatin, hydroxypropyl methyl cellulose or any mixture of the above vehicles.

Because of the negligible water-solubility of the capsaicinoids, they cannot be introduced into the bioadhesive vehicle by the usual method (by swelling in an aqueous phase), so the following special process must be employed.

The capsaicinoid is evenly dispersed in the bioadhesive vehicle by dissolving the suitable quantity of the capsaicinoid in an organic solvent or a mixture of such solvents that contains some water and which has a slightly swelling effect on the polymer - conveniently ethanol and water, diethyl ether and water. Then the bioadhesive vehicle is wetted by the solvent, it is homogenized by kneading, then it is dried and, after drying, the impregnated vehicle is ground to a suitable grain size.

### Molecular packaging (not part of the claimed scope alone)

The capsaicinoid active agent may be used without being prepared beforehand as it is disclosed above, but its introduction in a molecular envelope can be advantageous to control the release of the active agent. For the purpose of packaging, suitable coronaethers or cyclodextrins had to be found. These ensure a slow release of the active agent at the same time because the equilibrium between the inclusion complex and the starting materials shifts but slowly towards the latter. In one of the possible solutions, the beta-cyclodextrin complexes only form efficiently in an aqueous medium (because of the H-bridge bonds), but the capsaicinoids are hardly soluble in water. So, as a first problem to be solved, a solvent had to be found that, on the one hand, dissolved the capsaicinoids adequately (e.g. ethanol, n-propanol, i-propanol, acetone etc.), and, on the other hand, it was freely miscible with water. In order to achieve a good result in complex forming, the process has to be controlled by adding water to the mixture in such a way that the solution should remain near to the point of saturation with reference to the capsaicinoid. The organic medium should be diluted with water to such a degree only that the recrystallization of the beta-cyclodextrin and/or its dissolution may not occur to a significant extent.

Especially the alpha-, beta- and gamma-cyclodextrins could be applied, but even substituted (e.g. by hydroxypropyl) cyclodextrins could be made use of. For the purpose of molecular enveloping, beta-cyclodextrin is the most preferred.

### Forming dosage units

The pharmaceutical composition in the invention can be packaged in any solid form, where tablets (floated tablets) or capsules are preferred.

The tablets - after having been produced by any of the known tabletting methods - are finally subjected to a taste-masking process known from the literature.

If capsules are produced, they can be finally treated in an abrading drum using a scouring powder or powder mixture, which can be conveniently selected from monosaccharides, mixtures of monosaccharides, disaccharides, mixtures of disaccharides, sugar alcohols, mixtures of sugar alcohols, alkaline hydrogen carbonates, mixtures of alkaline hydrogen carbonates, or any mixture of these may be used. During the scouring process, the scouring agent is replaced by a fresh one several times, preferably three times, and any remains of the powder are removed from the surface of the capsules by a pneumatic treatment.

As a summary, it can be said that a pharmaceutical composition having the desired properties can be produced only by formulation techniques which are not obvious to a skilled person (i.e. an answer had to be found to the problem of ensuring low local concentrations of the active agent on the mucous membrane in a basically oxidizing and aqueous environment).

### EXPERIMENTAL PART

### Pharmacological experiments

The animal experiments connected with the administration of capsaicinoids in small doses, which have served as the base of present invention, are described in the following papers: Gy. Mózsik, Ome. Abdel Salam, J. Szolcsányi : Capsaicin Sensory Nerve in Gastric Mucosal Damage and Protection, Akadémiai Kiadó Budapest, 1997. Human studies with healthy volunteers in accordance with GCP (Good Clinical Practice) were carried out as described: Gy. Mózsik, J. Szolcsányi, I. Rácz: Gastroprotection induced by capsaicin in healthy human subjects, World J. Gastroenterology, 2005; 11:5180-5184.

In the experiments, capsaicinoids were found to have an excellent protective effect in dosage units of 100 to 1200 µg, or more preferably in 200 to 800 µg per dosage unit range. The best results were obtained with about 400 µg per dosage unit. The pharmaceutical composition was administered at least once and no more than three times a day.

In all the experiments, the NSAID compounds were used in the usual dosage as detailed above.

### Formulation Examples

### A) Realized examples

### Example 1 (reference example)

### Preparation of capsaicinoid in a talc vehicle

Into a 500 ml glass-stoppered round bottom flask, 100 g powdered talc of pharmaceutical quality and 251 ml ethanol base solution of 0.800 mg/ml are measured, the latter containing capsaicinoids in a mixture as laid down in USP 27. The suspension is homogenized in a rotary vacuum film-type evaporator (Büchi, rotation speed 20-30 rpm, 40 °C) without a vacuum, for 30 minutes; then, after flushing with an inert gas (e.g. N₂, CO₂, or Ar), the chamber is evacuated to -350 to -400 Hgmm pressure relative to the atmospheric pressure. The suspension is subjected to evaporation until the in-flowing flushing gas starts stirring up the solid substance in the flask. After it the vacuum is ceased. The preparation is then desiccated to steady mass at 40 °C, in a vacuum of 80-85% in a vacuum disk drying cabinet. After the substance has reached steady mass, it is analyzed as to capsaicinoid content (HPLC). Depending on the result of the analysis, the preparation is diluted to the desired value by adding some of the vehicle. Finally, applying any of the known processes, the composition - either alone or together with any of the desired accompanying active agents - is made into a combined, oral composition, for example, in the form of a tablet having a coating that prevents gas exchange, subjected to taste masking, or a floated tablet or capsule. Undesirable remains of capsaicinoids adhering to the surface of the capsules is removed as described in Example 11.

### Example 2 (reference example)

### Preparation of capsaicinoid in a talc vehicle using an oily carrier

Into a 500 ml glass-stoppered round bottom flask, three components are measured: 100 g powdered talc of pharmaceutical quality; 200 ml (c. 0.05 g/ml) of a solution of glycerol trioleate in ethyl acetate (Loba Feinchemie); 51 ml (4 mg/ml) of a solution carrying a mixture of capsaicinoids (USP 27) dissolved in ethanol. The suspension is homogenized in a rotary vacuum film-type evaporator (Büchi, rotation speed 20-30 rpm, 40 °C) without a vacuum, for 30 minutes; then, after flushing with an inert gas (e.g. N₂, CO₂, or Ar), the chamber is evacuated to -350 to -400 Hgmm pressure relative to the atmospheric pressure. The suspension is subjected to evaporation until the in-flowing flushing gas starts stirring up the solid substance in the flask. After it the vacuum is ceased. The preparation is then desiccated to steady mass at 40 °C, in a vacuum of 80-85% in a vacuum disk drying cabinet. After the substance has reached steady mass, it is analyzed as to capsaicinoid content (HPLC). Depending on the result of the analysis, the preparation is diluted to the desired value by adding talc prepared as above but containing no capsaicinoids. Finally, applying any of the known processes, the composition - either alone or together with any of the desired accompanying drugs - is made into a combined, oral composition, for example, in the form of a tablet having a coating that prevents gas exchange, subjected to taste masking, or a floated tablet or capsule. Undesirable remains of capsaicinoids adhering to the surface of the capsules is removed as described in Example 11.

### Example 3 (reference example)

### Preparation of capsaicinoid in a polyol vehicle

The procedure is the same as described in Example. 1, however, as a vehicle, instead of talc, powdered mannitol (or other polyalcohol), micro-crystalline cellulose (e.g. P-10), or any mixture of the vehicle materials (including talc) is applied.

### Example 4 (reference example)

### Preparation of capsaicinoid in a polyol vehicle with an oily carrier

The procedure is the same as described in Ex. 2, however, as a vehicle, instead of talc, powdered mannitol (or other polyol), micro-crystalline cellulose (e.g. P-10), or an optional mixture of the vehicle materials (including talc) are applied.

### Example 5 (reference example)

### Preparation of capsaicinoid in a talc vehicle by spraying

A coating machine (ERWEKA AR402) rigged out with a vaporizer and a gas flushing attachment is placed in a hood. The spray nozzle is adjusted so that the surface covered by the atomizer is greater by 15% than the surface of the material in the stationary dragee pan. The temperature of the coating pan is set at 40 °C (Infrarubin 2 x 250 W). 500 g talc of pharmaceutical quality is measured into the drum and - after suction has been provided - it is started at the slowest rate. The pump of the vaporizer (max. pressure 25 bar) is set to forward 5 ml/min of liquid. 252 ml of a solution containing capsaicinoids (USP 27, 4 mg/ml) in ethanol is vaporized in time periods as follows: the vaporization goes on for 10 minutes, then it is suspended, and the coating pan is flushed with an inert gas (CO₂) pre-heated to 40 °C for 12 minutes at a rate of 6-8 l/min. The flushing by the inert gas is terminated before starting the next vaporization period. Vaporization and flushing periods are alternating till all the liquid has been vaporized. After the last 2.0 ml of solution has been atomized, the inert-gas flushing is kept up for another 10 minutes. On termination of the flushing, the drum is stopped. The preparation is then desiccated to steady mass at 40 °C, in a vacuum of 80-85% in a vacuum disk drying cabinet. After the substance has reached steady mass, it is analyzed as to capsaicinoid contents (HPLC). Depending on the result of the analysis, the preparation is diluted to the desired value by adding some quantities of the vehicle. Finally, applying any of the known processes, the composition - either alone or together with any of the desired accompanying active agents - is made into a combined, oral composition, for example, in the form of a tablet having a coating that prevents gas exchange, subjected to taste masking, or a floated tablet or capsule. Undesirable remains of capsaicinoids, adhering to the surface of the capsules is, removed as described in Example 11.

### Example 6 (reference example)

### Preparation of capsaicinoid in a talc vehicle by spraying, applying an oily carrier

The procedure is the same as described in Example 5, however, the composition of the liquid to be vaporized (sprayed) is as follows: glycerol trioleate dissolved in ethyl acetate (400 ml, 0.125 mg/ml) (Loba Feinchemie) mixed with 51 ml of a solution of capsaicinoids (according to USP 27, 20 mg/ml) in ethanol. The duration of the inert-gas flushing between two periods of vaporization is 10 minutes. The final adjustment of the capsaicinoid concentration is done by adding a mixture of solvents as above but without capsaicinoids.

### Example 7 (reference example)

### Preparation of capsaicinoid in a polyol vehicle by spraying, applying an oily carrier

The procedure is the same as described in Example 6, however, instead of using talc as a vehicle, powdered mannitol (or other polyol), micro-crystalline cellulose (e.g. P-10), or any mixture of these vehicle materials (including talc) is used.

### Example 8 (reference example)

### Capsaicinoid introduced into beta-cyclodextrin

1.00 g beta-cyclodextrin is measured into a 25 ml glass-stoppered weighing bottle. A teflon covered magnetic stirring rod is placed into the vessel and 5.00 ml of a solution of capsaicinoids dissolved in (abs.) ethanol (20 mg/ml) is added. The heatable magnetic mixer is started; the speed of the stirring is adjusted between 80 to 120 rpm, while the temperature of the suspension is set to 30 °C. Stirring is continued for 30 minutes. After it, while the stirring is kept up, 5.00 ml distilled water is added dropwise; all the time attention should be paid that no local water concentration is formed, which might cause precipitation. After the water has been added, the stirring and the temperature are kept up for further 4 hours. At the end of the fourth hour, heating is terminated and the suspension is supplemented by a further 10 ml of distilled water in the way mentioned above. Then, the suspension is left to cool to room temperature and the stirring is kept up for further 4 hours. The stirring is terminated and the suspension is immediately filtered (MN 640 D). The solid material is rinsed with 5 ml of a mixture of ethanol and water (20 : 80 V/V) right on the filter and then it is sucked to dryness. The solid substance together with the filter paper is put in a vacuum disk drying cabinet, and it is desiccated to steady mass in a vacuum of 75-85% at 40 °C. The yield is 98.9% based on the HPLC analysis of the combined filtrate plus liquor with reference to the capsaicinoids bonded in the complex. The capsaicinoid-beta-cyclodextrin complex is diluted to the desired value by untreated beta-cyclodextrin. Finally, applying any of the known processes, the composition - either alone or together with any of the desired accompanying active agents - is made into a combined, oral composition, for example, in the form of a tablet having a coating that prevents gas exchange, subjected to taste masking, or a pearl or capsule. Undesirable remains of capsaicinoids adhering to the surface of the capsules is removed as described in Example 11.

### Example 9

### Preparation of capsaicinoids introduced into a bioadhesive polymer matrix

A 50 ml beaker is fitted onto an adjustable heating plate. The beaker, in turn, is rigged out with a variable-speed laboratory mixer that has a kneading paddle (KPG mixer motor). 10.0 g guar gum and 20 ml distilled water are measured into the beaker. The heating plate is set to 40 °C and the mixer is started. The polymer is left to swell for 60 minutes. Then 10.0 ml (4 mg/ml) of a solution - in (abs.) ethanol - containing a mixture of capsaicinoids as in USP 27 is added. The kneading is kept up for another 15 minutes, then it is suspended for 30 minutes. After it the homogenization is continued in kneading and resting phases - 10 and 30 minutes respectively - until, at the end of a resting phase, no precipitation of material is seen. After the last homogenization the composition is dried to steady mass in a vacuum disk drying cabinet at 40 °C, in a vacuum of 80-85%. Then the dried substance is ground to a grain size smaller than 100 µm. The desired concentration is set by adding some of the matrix that contains no active agent. Finally, applying any of the known processes, the capsaicinoid introduced into the polymer matrix - either alone or together with any of the desired accompanying drugs - is made into a combined, oral composition, for example, in the form of a tablet having a coating that prevents gas exchange, subjected to taste masking, or floated tablet or capsule. Undesirable remains of capsaicinoids adhering to the surface of the capsules is removed as described in Example 11.

### Example 10

The procedure is the same as described in Example 9, but as bioadhesive polymer the followings are applied: carboxymethyl cellulose-Na*, Na-alginate*, hydroxypropyl methyl cellulose, polyvinyl pyrrolidon K-30*, polyvinyl pyrrolidon K-90*, gelatin, or any mixture of these. The quantity of water is measured in such a way that at the end of the 60th minute the polymer is not crumbly but forms a consistent, kneadable mass.
* not part of the claimed scope

### Example 11

### Dedusting of the capsules

1000 capsules and 1000 g powdered glucose are loaded into a coating pan. The speed of the pan is set at 35 to 50 rpm and the dedusting process is kept up for 10 minutes. The contents of the pan is poured onto a sieve with a 2.5 mm mesh (sieve No. DIN 1171, 2.5). The capsules and the powder are separated; any remaining powder is further cleaned off by applying a stream of gas while the capsules are still on the sieve. The previous sequence of actions is repeated twice using fresh glucose powder. In the fourth (last) dedusting run 1000 g powdered mannitol is used instead of glucose. After it the capsules are subjected to final packaging. The glucose separated during the third dedusting round is used again in the first run with the next batch. The mannitol can be used three more times.

### B) Theoretical examples

In this section theoretical examples are described that demonstrate the feasibility of the combined pharmaceutical compositions according to the invention, which have not yet been realized because of the difficulties of the field of authorization of experiments on human drug applications, but - to the best of our knowledge - they support the applicability and functionality of the invention. The scope of protection of the invention is, of course, not limited to the actual embodiments described herein.

### Theoretical example 1

In the long-term treatment of patients taking one aspirin (tablet or capsule) orally a day, besides the daily dose of aspirin (100 or 500 mg) a capsaicinoid supplement (400 µg) is orally administered in a combined composition of aspirin plus capsaicinoid. In the case of patients who are aspirin resistant, the following is recommended: 300 mg aspirin plus 400 µg capsaicinoid, orally, once a day, for a long term.

### Theoretical example 2

With patients taking diclofenac - 25, 50 or 75 mg diclofenac in a tablet, taken orally twice a day - alongside each dosage 400 µg capsaicinoid is orally administered in a combined composition of diclofenac plus capsaicinoid. It could be taken occasionally (as a painkiller), or for a short or long term (for weeks).

### Theoretical example 3

With patients taking naproxen - 25 mg naproxen in a tablet, taken orally twice a day - alongside each dosage 400 µg capsaicinoid is orally administered in a combined composition of naproxen plus capsaicinoid. This treatment may be fruitful with patients suffering from cardiological and/or chronic, degenerative locomotory organ diseases (anti-inflammatory or palliative effect).

### Theoretical example 4 (reference example)

The product obtained in Example 8 above, is undertaken to an anti-oxidant treatment as described in Example 2 or Example 6, with the exception that the oily protecting layer is formed on the surface of 100 g of cyclodextrin-capsaicinoid inclusion complex; the amount of ethyl acetate used in the examples is reduced by half while that of the oily protective substance is unchanged.

### Theoretical example 5

The product having a suitable grain size obtained in Example 4, or the product having a suitable grain size obtained in Example 8 is introduced into a bioadhesive polymer matrix analogously with Example 9, but with the following difference:
- the chosen bioadhesive polymer matrix (which can be any mixture of bioadhesive polymers) is made kneadable by the addition of distilled water;
- after adding the products obtained in Theoretical Example 4 or Example 8, it is homogenized by swelling and stirring;
- the obtained homogenous mixture is dried and worked up analogously with Example 9.

### Notes to the examples above:

As seen in the theoretical examples above, the capsaicinoid is in each case given in a dose of 400 µg with the non-steroidal anti-inflammatory drug (aspirin, diclofenac, naproxen). Capsaicinoids are administered in doses 400 to 800 µg per patient per day, given once or twice a day. The small dose of capsaicinoid may vary from 100 to 1200 µg, preferably at least 200 µg, more preferably 400 µg per dosage unit, administered once a day or repeated a second time adjusted to the administration of the NSAID.

The compositions - as a result of the special vehicle materials and the introduction of the active agent into them - described in the above examples make it possible for the capsaicinoid to get released locally, directly on the mucous membrane. That is exactly how the locally beneficial, stomach-protecting effect can be achieved using small doses of capsaicinoids, without causing systemic effects at the same time.

As the combined composition according to the invention diminishes or protects against the gastro-intestinal side effects of any non-steroidal anti-inflammatory drugs, it could be used in any therapy where such anti-inflammatory agents are administered orally, so it may be indicated in the following conditions:
- gastro-intestinal inflammatory conditions (gastritis, helicobacter positive or negative clinical appearance),
- gastro-intestinal inflammatory pathography (precancerous conditions, tumorous conditions accompanying the significant increase of COX-2 activity),
- conditions resulting from the proliferation of oxygen free radicals,
- irritable intestinal, or chronic abdominal pain syndromes.

Furthermore, the combined composition according to the invention may be used in the following cases:
- protecting against or diminishing the side effects of oral treatment in chronic cases with non-steroidal drugs, especially with salicylic acid or its derivatives (e.g. cardiovascular conditions, stroke, inflammatory diseases of the organs of locomotion and of the lower limbs),
- patients suffering a cardiac infarct or being prior to an anticipated a vascular catastrophe (stroke), who are treated against blood clotting for a long term with aspirin with daily preventive dose of 100 mg, in aspirin-resistant patients of 300 mg, as recommended by the American and European Cardiological Societies, for protecting against or diminishing the side effects.

The amount of the NSAID that is necessary to achieve the desired therapeutic effect depends, in the first place, on the compound actually used, but, besides, on several further factors, i.e. the method of administering the drug, the age and condition of the patient. With aspirin, the usual human dose is about 50 to 500 mg (in the treatment or follow-up care of cardiological patients 100-300 mg per patient per day), or when taken long-term, 100 to 300 mg per patient per day. With diclofenac, the dose varies between 25x2 mg, 50x2 mg, 75x2 mg per patient per day, while, with naproxen, it is 250 mg, 375 mg, or 500 mg per patient per day.

The desirable amount of capsaicinoid to accompany a dose unit of one of the non-steroidal agents is about 100 to 1200 µg per dosage unit, preferably at least 200 µg, preferably 200 to 800 µg per dosage unit, more preferably about 400 µg per dosage unit.

The production of capsaicinoid is a well-known process but it is also available commercially, e.g. from Ashian Herbex Ltd (Ahra Pades India).

### Advantages

The combined pharmaceutical composition according to the invention besides diminishing the gastro-intestinal side effects of the NSAID - through a special protecting mechanism exerted by the small-dose capsaicinoids - can also protect against any damage to the human stomach and intestines caused by any internal or external effect (chemical, physical, bacterial etc.).

## Claims

1. A combined solid, oral pharmaceutical composition for use in diminishing or protecting against the gastro-intestinal side effects of non-steroidal anti-inflammatory agents administered orally in a therapy, which contains a capsaicinoid active agent in a dose of 100 to 1200 µg per dosage unit, one or more non-steroidal anti-inflammatory active agent(s) and one or more usual pharmaceutical auxiliary materials, ***characterized in that*** the composition comprises the active agents and other ingredients in a vehicle system which comprises
one or more bioadhesive polymer(s) selected from the goup comprising guar gum, gelatin, hydroxypropyl methyl cellulose or any mixtures thereof.

2. The pharmaceutical composition for use according to claim 1, wherein the bioadhesive polymer vehicle is guar gum.

3. The pharmaceutical composition for use according to any of claims 1 to 2, wherein the non-steroidal anti-inflammatory agent is diclofenac, preferably in 25 to 75 mg per dosage unit, or aspirin, preferably in 50 to 500 mg per dosage unit, or naproxen, preferably in 250 to 500 mg per dosage unit.

4. The pharmaceutical composition for use according to any of claims 1 to 3, wherein the dose of capsaicinoid is 200 to 800 µg per dosage unit, preferably 400 µg per dosage unit.

5. The pharmaceutical composition for use according to any of claims 1 to 4 which contains as usual vehicle talc, micro-crystalline cellulose, C₄₋₇ sugar or sugar alcohol, e.g. mannitol, or any mixtures thereof.

6. A combined solid, oral pharmaceutical composition for use in diminishing or protecting against the gastro-intestinal side effects of non-steroidal anti-inflammatory agents administered orally in a therapy, which comprises in a combined packaging, preferably in a twin blister
A) a separate, first pharmaceutical composition, preferably a tablet or capsule, which contains one or more non-steroidal anti-inflammatory active agent(s), together with usual pharmaceutical auxiliary materials and/or carriers; and
B) a separate, second pharmaceutical composition according to any of claims 1 to 5 without non-steroidal anti-inflammatory active agent.

7. A solid, oral pharmaceutical composition for use according to claim 6, wherein the non-steroidal anti-inflammatory agent is diclofenac, preferably in 25 to 75 mg per dosage unit, or aspirin, preferably in 50 to 500 mg per dosage unit, or naproxen, preferably in 250 to 500 mg per dosage unit.

## Patentansprüche

1. Eine kombinierte feste orale pharmazeutische Zusammensetzung für die Verwendung bei der Verringerung von oder beim Schutz vor gastrointestinalen Nebenwirkungen von nicht-steroidalen entzündungshemmenden Mitteln, die in einer Therapie oral verabreicht werden, welche einen capsaicinoiden Wirkstoff in einer Dosis von 100 bis 1200 µg per Dosiseinheit, einen oder mehreren nicht-steroidalen entzündungshemmenden Wirkstoffe und einen oder mehreren üblichen pharmazeutischen Hilfsstoffe enthält, ***dadurch gekennzeichnet, dass*** die Zusammensetzung die Wirkstoffe und andere Bestandteile in einem Trägersystem enthält, welches
ein oder mehrere bioadhesiven Polymere ausgewählt aus der Gruppe enthaltend Guargummi, Gelatine, Hydroxypropyl-methyl-cellulose oder beliebige Mischungen davon, enthält.

2. Die pharmazeutische Zusammensetzung für die Verwendung nach Anspruch 1, worin der bioadhesive Polymer-Trägerstoff Guargummi ist.

3. Die pharmazeutische Zusammensetzung für die Verwendung nach einem der Ansprüche 1 bis 2, worin das nicht-steroidale entzündungshemmende Mittel Diclofenac, bevorzugt in einer Dosis von 25 bis 75 mg per Dosiseinheit, oder Aspirin, bevorzugt in einer Dosis von 50 bis 500 mg per Dosiseinheit, oder Naproxen, bevorzugt in einer Dosis von 250 bis 500 mg per Dosiseinheit, ist.

4. Die pharmazeutische Zusammensetzung für die Verwendung nach einem der Ansprüche 1 bis 3, worin die Dosis von Capsaicinoid 200 bis 800 µg per Dosiseinheit, bevorzugt 400 µg per Dosiseinheit, beträgt.

5. Die pharmazeutische Zusammensetzung für die Verwendung nach einem der Ansprüche 1 bis 4 welche als üblichen Trägerstoff Talkum, mikrokristalline Cellulose, C₄₋₇-Zucker oder Zuckeralkohol, z.B. Mannitol, oder beliebige Mischungen davon enthält.

6. Eine kombinierte feste orale pharmazeutische Zusammensetzung für die Verwendung bei der Verringerung von oder beim Schutz vor gastrointestinalen Nebenwirkungen von nicht-steroidalen entzündungshemmenden Mitteln, die in einer Therapie oral verabreicht werden, welche in einer kombinierten Packung, bevorzugt in einer doppelten Blisterverpackung
A) eine gesonderte erste pharmazeutische Zusammensetzung, bevorzugt eine Tablette oder Kapsel, welche einen oder mehrere nicht-steroidale entzündungshemmende Wirkstoffe zusammen mit einem oder mehreren üblichen pharmazeutischen Hilfsstoffen und/oder Trägerstoffen enthält; und
B) eine gesonderte zweite pharmazeutische Zusammensetzung nach einem der Ansprüche 1 bis 5 ohne nicht-steroidalen entzündungshemmenden Wirkstoff enthält,
enthält.

7. Die feste orale pharmazeutische Zusammensetzung für die Verwendung nach Anspruch 6, worin das nicht-steroidale entzündungshemmende Mittel Diclofenac, bevorzugt in einer Dosis von 25 bis 75 mg per Dosiseinheit, oder Aspirin, bevorzugt in einer Dosis von 50 bis 500 mg per Dosiseinheit, oder Naproxen, bevorzugt in einer Dosis von 250 bis 500 mg per Dosiseinheit, ist.

## Revendications

1. Composition pharmaceutique orale solide combinée pour son utilisation pour réduire ou protéger contre les effets secondaires gastro-intestinaux des agents anti-inflammatoires non stéroïdiens administrés par voie orale dans une thérapie, qui contient un agent capsaïcinoïde actif à une dose de 100 à 1200 µg par unité posologique, un ou plusieurs agents anti-inflammatoires non stéroïdiens actifs et un ou plusieurs auxiliaires pharmaceutiques habituels, **caractérisé en ce que** la composition comprend les agents actifs et d'autres ingrédients dans un système de véhicule qui comprend
un ou plusieurs polymère(s) bioadhésif(s) choisi(s) dans le groupe comprenant la gomme de guar, la gélatine, l'hydroxypropylméthylcellulose ou l'un quelconque de leurs mélanges.

2. Composition pharmaceutique pour son utilisation selon la revendication 1, dans laquelle le véhicule polymère bioadhésif est la gomme de guar.

3. Composition pharmaceutique pour son utilisation selon l'une quelconque des revendications 1 à 2, dans laquelle l'agent anti-inflammatoire non stéroïdien est le diclofénac, de préférence de 25 à 75 mg par unité posologique, ou l'aspirine, de préférence de 50 à 500 mg par unité posologique, ou le naproxène, de préférence de 250 à 500 mg par unité posologique.

4. Composition pharmaceutique pour son utilisation selon l'une quelconque des revendications 1 à 3, dans laquelle la dose de capsaïcinoïde est de 200 à 800 µg par unité posologique, de préférence de 400 µg par unité posologique.

5. Composition pharmaceutique pour son utilisation selon l'une quelconque des revendications 1 à 4, qui contient comme véhicule habituel du talc, de la cellulose microcristalline, du sucre ou de l'alcool de sucre en C₄₋₇, par ex. le mannitol, ou l'un quelconque de leurs mélanges.

6. Composition pharmaceutique orale solide combinée pour son utilisation pour réduire ou protéger contre les effets secondaires gastro-intestinaux des agents anti-inflammatoires non stéroïdiens administrés par voie orale dans une thérapie, qui comprend dans un emballage combiné, de préférence dans une double coque
A) une première composition pharmaceutique séparée, de préférence un comprimé ou une capsule, qui contient un ou plusieurs agents anti-inflammatoires non stéroïdiens actifs, ainsi que des auxiliaires et/ou des supports pharmaceutiques habituels ; et
B) une seconde composition pharmaceutique séparée selon l'une quelconque des revendications 1 à 5 sans agent anti-inflammatoire non stéroïdien actif.

7. Composition pharmaceutique orale solide pour son utilisation selon la revendication 6, dans laquelle l'agent anti-inflammatoire non stéroïdien est le diclofénac, de préférence de 25 à 75 mg par unité posologique, ou l'aspirine, de préférence de 50 à 500 mg par unité posologique, ou le naproxène, de préférence de 250 à 500 mg par unité posologique.
